(19) Europäisches Patentamt
European Patent Office
Office européen des brevets

(11) EP 0 556 395 B1

(12) # EUROPEAN PATENT SPECIFICATION

(45) Date of publication and mention of the grant of the patent:
**01.10.2003 Bulletin 2003/40**

(21) Application number: **91919165.0**

(22) Date of filing: **07.11.1991**

(51) Int Cl.[7]: **A61K 38/00**, C07K 14/00, C12N 15/24, C12N 5/10, C12P 21/02

(86) International application number:
**PCT/JP91/01526**

(87) International publication number:
**WO 92/008735 (29.05.1992 Gazette 1992/12)**

(54) **NOVEL CYTOKINE**

NEUARTIGE CYTOKINE

NOUVELLE CYTOKINE

(84) Designated Contracting States:
**AT BE CH DE DK ES FR GB GR IT LI LU NL SE**

(30) Priority: **08.11.1990 JP 30355890**
**29.01.1991 JP 878591**

(43) Date of publication of application:
**25.08.1993 Bulletin 1993/34**

(73) Proprietor: **SANKYO COMPANY LIMITED**
**Chuo-ku, Tokyo 103-0023 (JP)**

(72) Inventors:
- **KAWASHIMA, Ichiro, c/o Sankyo Company, Ltd. Tokyo 140 (JP)**
- **OHSUMI, Jun, c/o Sankyo Company, Ltd. Tokyo 140 (JP)**
- **MIYADAI, Kenji, c/o Sankyo Company, Ltd. Tokyo 140 (JP)**
- **TAKIGUCHI, Yo, c/o Sankyo Company, Ltd. Tokyo 140 (JP)**

(74) Representative:
**Gibson, Christian John Robert et al**
**MARKS & CLERK,**
**57/60 Lincoln's Inn Fields**
**London WC2A 3LS (GB)**

(56) References cited:
**WO-A-91/07495**

- **LYMPHOKINE RESEARCH vol. 9, no. 4 , October 1990 page 601 J. GIANNOTTI ET AL 'Biologic properties of recombinant human Interleukin-11 , a novel multi-functional stromal cell-derived cytokine' & THE SEVENTH INTERNATIONAL LYMPHOKINE WORKSHOP , 1-5 OCTOBER 1990 . SAN ANTONIO, TEXAS; USA**
- **Proceedings of the National Academy of Sciences, U.S.A., Vol. 87, No. 19, (1990), S.R. PAUL et al. "Molecular cloning of a cDNA encoding intevleukin 11, a stromal cell-derived lymphopoietic and hematopoietic cytokine" p. 7512-7516.**
- **FEBS Letters, Vol. 283, (1991, I. KAWASHIMA et al. "Molecular cloning of complementary DNA encoding adipogenesis inhibitory factor and indentity with interleukin-11" p. 199-202.**
- **FEBS Letters, Vol. 288, (1991), J. OHSUMI et al. "Adipogenesis inhibitory factor. A novel inhibitory regulator of adipose conversion in bone marrow" p. 13-16.**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 556 395 B1

## Description

[Technical Field]

The present invention relates to a novel single protein which is capable of suppressing differentiation of cells into adipocytes, capable of suppressing lipoprotein lipase (LPL) in adipocytes and/or which is capable of inducing colony stimulating factor (CSF). The present invention also relates to DNA that encodes said protein, a recombinant DNA expression vector comprising said DNA, a host cell that is transformed by said recombinant DNA expression vector, and a protein composition including an effective amount of said protein.

[Background Art]

It is widely known that the existence of bone marrow stromal cells that support hematopoiesis, in addition to hematopoietic stem cells and various hematopoietic factors, or in other words, a hematopoietic microenvironment, is necessary for hematopoiesis in a living body. In humans, at the time of birth, hematopoiesis actively takes place in bone marrow throughout the body. However, when hematopoiesis no longer becomes necessary as he or she grows up, the bone marrow gradually becomes occupied by adipocytes, starting with the extremities of the limbs due to enlargement of medullary space. Bone marrow in which hematopoiesis is taking place is referred to as red marrow, while bone marrow which has been replaced by adipocytes is referred to as yellow marrow. When hematopoiesis is accelerated due to bleeding or hemolysis, hematopoiesis resumes in the yellow marrow [Kashiwamura, M. (1988), "IGAKU NO AYUMI (History of Medicine)", 146, 264-268].

Cells which have a hematopoietic function in the hematopoietic microenvironment are primarily preadipocytes. It is known that, when these cells differentiate into adipocytes, they lose this function [Kodama, H. (1986), "SOSHIKI BAIYO (Tissue Culture)", 12, 191-195].

For example, mouse bone marrow-type preadipocyte cell line PA6 is capable of supporting the proliferation of hematopoietic stem cells. However, when these cells differentiate into adipocytes, this activity diminishes [Kodama, H. et al. (1984), J. Cell. Physiol. 118, 233-240]. It is known that blast cell, megakaryocyte and macrophage colonies can be induced when PA6 cells and mouse bone marrow cells are cultured together [Kodama, H. (1987), "JIKKEN IGAKU" (Experimental Medicine)", 5, 826-830]. Thus, protein capable of suppressing adipogenesis acts on the preadipocytes and the adipocytes of bone marrow to promote their hematopoietic ability, or in other words, promote their ability to induce leukocytes, macrophages and platelets (originating in megakaryocytes).

In addition, it is reported that the amount of CSF produced in the preaidpocyte cell line H-1/A, originating from mouse bone marrow, is reduced when these cells differentiate into adipocytes [Nakamura, M. et al. (1985), Proc. Soc. Exp. Biol. Med., 179, 283-287].

As will be described below, the adipogenesis inhibitory factor of the present invention acts on H-1/A cells to accelerate production of macrophage colony stimulating factor (M-CSF). In addition to stimulative activity against monocytes and macrophages, M-CSF is also capable of acting as a megakaryocyte potentiator (Meg-POT) [Teramura, M. et al. (1990), Int. J. Cell Cloning, 8, 245-252]. M-CSF is also known to demonstrate an ability to act on monocytes to accelerate their production of Meg-POT. Moreover, data from clinical therapy indicate that M-CSF promotes recovery from neutropenia and thrombocytopenia following cancer chemotherapy [Motoyoshi, K. (1986), "GAN TO KAGAKURYOHO (Cancer and Chemotherapy)", 16, 3531-3536].

Thus, the adipogenesis inhibitory factor of the present invention acts on bone marrow or peripheral preadipocytes and adipocytes to enhance their ability to support hematopoiesis.

Examples of proteins, known prior to the present invention and which suppress adipocyte differentiation and LPL in adipocytes, include: tumor necrosis factor-$\alpha$ (TNF-$\alpha$) [Price, S.R. et al. (1986), Arch. Biochem. Biophys., 251, 738-746 and Kawakami, M. et al. (1987), J. Biochem., 101, 331-338]; interleukin-1 (1L-1) [Beutler, B.A. and Cerami, A. (1985), J. Immunol., 135, 3969-3971]; interferons (IFN) [Keay, S. and Grossberg, S.E. (1980), Proc. Natl. Acad. Sci. USA, 77, 4099-4103, and Patton, J.S. et al. (1986), Proc. Natl. Acad. Sci. USA, 83, 8313-8317]; and leukemia inhibitory factor (LIF) [Mori, M. et al. (1989), Biochem. Biophys. Res. Commun., 160, 1085-1092]. The cDNAs of these factors have already been cloned, and their entire structures confirmed.

In addition, although a report was published prior to the priority date of the present invention describing the isolation and expression of cDNA of the precursor of monkey interleukin-11, along with the discovery of its plasmacytoma growth stimulating activity and megakaryocyte colony formation activity in the culture of human interleukin-11.

US07/441,100 (abandoned) and-US07/526,474 (published as US-A-5 215 895) both disclose interleukin 11 precursor sequences, but neither disclose the mature IL-11 sequence.

The present inventors have discovered a novel single protein primarily capable of: suppressing differentiation of cells into adipocytes; suppressing lipoprotein lipase (LPL) in adipocytes; and inducing colony stimulating factor (CSF) (CSF inducing factor), and have made it possible to obtain large amounts of said protein using genetic manipulation techniques.

The various activities comprising: suppressing morphological differentiation of preadipocyte cells into adipocytes; suppressing lipoprotein lipase (LPL) in adipocytes; and maintaining and promoting the ability of preadipocytes to produce CSF, may individually or jointly be referred to as "adipogenesis inhibitory activity", and a protein having such activity may be referred to as an "adipogenesis inhibitory factor".

The present invention permits the collection of large amounts of adipogenesis inhibitory factor. As such, the factor can effectively be used in the treatment and diagnosis of various types of anaemias and other blood diseases. For example, the factor can be applied in aplastic anaemia, leukopaenia caused by toxins or radiation, infections caused by viruses, bacteria and parasites, cytopaenia occurring after a bone marrow transplant, and cytopaenia caused by chemotherapy with carcinostatics. In addition, the factor can also enable the preservation of blood transfusion components as presently performed on a wide scale. Moreover, in addition to these immunocompetence enhancing effects, the factor can also be used in the prevention of morbid obesity, or as a therapeutic preparation for disease. prevention of morbid obesity, or as a therapeutic preparation for disease.

"Cytopaenia", as used in the present invention, pertains to all of these diseases, as well as to various incidental immune diseases. "Cytopaenia ameliorant" is used as a generic name for those drugs effective in the prevention and treatment of cytopaenia.

In a first aspect, the present invention provides a protein that includes no other human originating proteins, and has the amino acid sequence of amino acid numbers 1 to 178 of that indicated in SEQ. ID. NO. 2 of the sequence listing, or wherein one or more of the amino acid residues are deleted at one or more sites of said protein or wherein one of the amino acid residues is substituted at one of the sites of said protein, the protein being a 178 mer or part thereof, wherein the protein as part thereof possesses adipogenesis inhibitory activity.

More preferably, the present invention provides a protein that includes no other human originating proteins, consists of the amino acid sequence 1-178 of SEQ ID NO: 2 of the sequence listing and possesses adipogenesis inhibitory activity.

The present invention further provides DNA encoding the proteins, especially the preferred protein, of the invention. Further, DNA encoding the proteins is provided, consisting of the nucleotide sequence 81 to 614 of SEQ ID NO: 1 of the sequence listing.

The present invention yet further provides a recombinant DNA expression vector comprising the DNA defined above, wherein the DNA can be expressed and replicated, as well as providing a host cell transformed by such a vector.

The present invention still further provides a process for producing a protein, which comprises: culturing a host cell transformed by a vector as defined above, and recovering the protein from the cell extract or the culture solution.

Also provided is a pharmaceutical composition containing a therapeutically effective amount of the protein of the present invention, together with a pharmaceutically acceptable carrier or vehicle.

Particularly preferred is a cytopaenia ameliorant containing a therapeutically effective amount of the protein of the present invention, together with a pharmaceutically acceptable carrier or vehicle.

Also preferred is an anti-obesity preparation containing a therapeutically effective amount of the protein of the present invention, together with a pharmaceutically acceptable carrier or vehicle.

The present invention, in addition, provides use of a protein as defined above in the preparation of a medicament for cytopaenia and/or obesity.

As described above, the N terminal amino acid sequence of the proteins of the present invention are amino acids 1-10 of SEQ ID NO: 2, i.e. (N)-Pro Gly Pro Pro Pro Gly Pro Pro Arg Val.

The amino acid sequence (amino acids 1 - 178 of SEQ ID NO: 2) referred to above is as follows:

```
(N)
Pro Gly Pro Pro Pro Gly Pro Pro Arg Val Ser Pro Asp Pro Arg
Ala Glu Leu Asp Ser Thr Val Leu Leu Thr Arg Ser Leu Leu Ala
Asp Thr Arg Gln Leu Ala Ala Gln Leu Arg Asp Lys Phe Pro Ala
Asp Gly Asp His Asn Leu Asp Ser Leu Pro Thr Leu Ala Met Ser
Ala Gly Ala Leu Gly Ala Leu Gln Leu Pro Gly Val Leu Thr Arg
Leu Arg Ala Asp Leu Leu Ser Tyr Leu Arg His Val Gln Trp Leu
```

Arg Arg Ala Gly Gly Ser Ser Leu Lys Thr Leu Glu Pro Glu Leu
Gly Thr Leu Gln Ala Arg Leu Asp Arg Leu Leu Arg Arg Leu Gln
Leu Leu Met Ser Arg Leu Ala Leu Pro Gln Pro Pro Pro Asp Pro
Pro Ala Pro Pro Leu Ala Pro Pro Ser Ser Ala Trp Gly Gly Ile
Arg Ala Ala His Ala Ile Leu Gly Gly Leu His Leu Thr Leu Asp
Trp Ala Val Arg Gly Leu Leu Leu Leu Lys Thr Arg Leu-(C)

The nucleotide sequence (81 to 614 of SEQ ID NO: 1) referred to above is as follows:

(5')- CCT GGG CCA CCA
CCT GGC CCC CCT CGA GTT TCC CCA GAC CCT CGG GCC GAG CTG
GAC AGC ACC GTG CTG CTG ACC CGC TCT CTC CTG GCG GAC ACG
CGG CAG CTG GCT GCA CAG CTG AGG GAC AAA TTC CCA GCT GAC
GGG GAC CAC AAC CTG GAT TCC CTG CCC ACC CTG GCC ATG AGT
GCG GGG GCA CTG GGA GCT CTA CAG CTC CCA GGT GTG CTG ACA
AGG CTG CGA GCG GAC CTA CTG TCC TAC CTG CGG CAC GTG CAG
TGG CTG CGC CGG GCA GGT GGC TCT TCC CTG AAG ACC CTG GAG
CCC GAG CTG GGC ACC CTG CAG GCC CGA CTG GAC CGG CTG CTG
CGC CGG CTG CAG CTC CTG ATG TCC CGC CTG GCC CTG CCC CAG
CCA CCC CCG GAC CCG CCG GCG CCC CCG CTG GCG CCC CCC TCC
TCA GCC TGG GGG GGC ATC AGG GCC GCC CAC GCC ATC CTG GGG
GGG CTG CAC CTG ACA CTT GAC TGG GCC GTG AGG GGA CTG CTG
CTG CTG AAG ACT CGG CTG -(3')

The DNA of the present invention is obtained, for example, by preparing mRNA coding for a protein possessing adipogenesis inhibitory activity, from mammalian cells which have the capacity to produce the protein, followed by reverse transcription of the mRNA into double-stranded cDNA by known methods. Cell line KM-102 of human bone marrow stromal origin [Harigaya, K. and Handa, H. (1985), Proc. Natl. Acad. Sci. USA, 82, 3477-3480] is a preferred source of animal cell mRNA, but other tumour cell strains and cells and tissue that can be isolated from mammals, or other established cell lines, may also be used.

The guanidine thiocyanate hot phenol method and the guanidine thiocyanate-guanidine hydrochloric acid method can be employed in the extraction of mRNA, but the guanidine thiocyanate caesium chloride method is preferred.

The majority of mRNA present in the cytoplasm of eukaryotic cells has a poly-A tail at the 3' terminal, so that mRNA may be adsorbed onto an oligo(dT) cellulose column to take advantage of this characteristic, and the mRNA may then be purified by elution. This mRNA can further be fractionated by methods such as sucrose density gradient centrifugation.

In order to confirm that the protein encoded by the mRNA obtained above has adipogenesis inhibitory activity, the mRNA can be translated. The resulting protein may then be assayed for physiological activity, or identified using an antibody specific for the protein. Translation into protein may be effected, for example, by injecting mRNA into African tree frog (Xenopus laevis) oocytes [Gurdon, J.B. et al. (1972), Nature, 233, 177-182]. Other translation systems include the rabbit reticulocyte lysate system and the wheat germ extract system [Schleif, R.F. and Wensink, P.C. (1981), "Practical Methods in Molecular Biology", Springer-Verlag, NY].

Adipogenesis inhibitory activity can be assayed by measuring the ability of the protein to suppress the differentiation of preadipocytes into adipocytes using, for example, mouse cell line 3T3-L1, or measuring LPL activity [Beutler, B.A. et al. (1985), J. Immunol., 135, 3972-3977].

Single-stranded cDNA can be synthesised from the thus obtained mRNA by a reverse transcriptase, and double-stranded cDNA can then be synthesised, using the single-stranded cDNA as a template. Suitable methods which can be used for this synthesis include: the S1 nuclease method [Efstratiadis, A. et al. (1976), Cell, 7, 279-288]; the Land method [Land, H. et al. (1981), Nucleic Acids Res., 9, 2251-2266]; and the O. Joon Yoo method [Yoo, O.J. et al. (1983), Proc. Natl. Acad. Sci. USA, 79, 1049-1053] . However, the preferred method is that of Okayama-Berg [Okayama, H. and Berg, P. (1982), Mol. Cell. Biol., 2, 161-170] , for the purposes of the present invention.

The resulting recombinant plasmids may then be introduced into Escherichia coli, for example the DH5 $\alpha$ strain, to obtain a transformed host. Desired recombinants can be selected using tetracyclin resistance or ampicillin resistance as an index. For example, in the case where the host cell is Escherichia coli, transformation can be performed using the Hanahan method [Hanahan, D. (1983), J. Mol. Biol., 166, 557-580]. More specifically, transformation can be carried out by introduction of the recombinant DNA to competent cells prepared with $CaCl_2$ , $MgCl_2$ or RbCl. Incidentally, lamda 'phage, for example, can also be used as vectors, instead of plasmids.

The various methods described below can be employed for selection of a strain possessing DNA coding for the desired adipogenesis inhibitory factor from the transformed host.

(1) Screening Method Using Synthetic Oligonucleotide Probe

An oligonucleotide probe may be synthesised where the amino acid sequence of the desired protein has been elucidated, either in whole or in part (this may correspond to any region of the desired protein if the sequence consists of a plurality of continuing specific sequences), and which codes for a part of the known sequence. The nucleotide sequence may be designed based on the frequency of codon usage, or a plurality of probes may be synthesised each of which has a different sequence encoding the same amino acid sequence. In the latter case, the number of sequences can be minimised by incorporating inosine into the sequence.

The resulting oligonucleotide can then be used as a probe (labelled with $^{32}$P or $^{35}$S) to hybridise with the denatured DNA of the transformed strain, after the DNA has been immobilised on a nitrocellulose filter, followed by screening and selection of the resulting positive strain.

(2) Screening Using a Probe Prepared by Polymerase Chain Reaction

In the case where the amino acid sequence of the target protein has been totally or partially elucidated, oligonucleotides of the sense and anti-sense strands, corresponding to a portion of amino acid sequence, may be synthesised. A polymerase chain reaction [Saiki, R.K. et al. (1988), Science, 239, 487-491] can be performed using these oligonucleotides to amplify the DNA fragment coding for the desired adipogenesis inhibitory factor. cDNA synthesised from genome DNA or by reverse transcription from mRNA of cells that produce adipogenesis inhibitory factor, can be used as the template DNA in this process. After labelling of the thus prepared DNA fragment with $^{32}$P or $^{35}$S, the desired clone is selected by performing colony hybridisation or plaque hybridisation using the labelled DNA fragment as the probe.

(3) Screening by Producing Adipogenesis Inhibitory Factor Using Other Animal Cells

Strains having cDNA coding for the desired adipogenesis inhibitory factor can be selected from the original transformed cell strains by culturing the transformed strain, amplifying the gene, transfecting the gene into animal cells (in this case, either a self-replicatable plasmid containing a transcription promoter region or a plasmid which can be integrated into an animal cell chromosome), expressing the protein coded by the gene and, either measuring the adipogenesis inhibitory activity of the culture supernatant or cell extract, or detecting adipogenesis inhibitory factor using an antibody for the factor.

(4) Selection Using Antibody for Adipogenesis Inhibitory Factor

The desired strain is selected by inserting cDNA into an expression vector in advance, producing protein within the transformant, and detecting the transformant producing the desired adipogenesis inhibitory factor using an antibody for the adipogenesis inhibitory factor and a secondary antibody for the antibody.

(5) Using a Selective Hybridization Translation System

In this method, mRNA from cells producing adipogenesis inhibitory factor is hybridised to cDNA obtained from the transformant, the cDNA having been blotted onto a nitrocellulose filter or the like. Subsequently, bound mRNA is dissociated and recovered. The recovered mRNA is then either injected into a protein translation system, such as African tree frog oocytes, or translated into a protein using a cell-free system, such as rabbit reticulocyte lysate or wheat germ extract, to examine the adipogenesis inhibitory activity of the protein or to detect adipogenesis inhibitory factor using an antibody for the factor.

As described in the following Examples, the desired transformant can also be selected by applying the above method (3) for the selected positive transformants, after a primary screening has been performed using a synthetic oligonucleotide probe designed with reference to the AUUUA motif [Shaw, G. and Kamen, R. (1986), Cell, 46, 659-667] present in the mRNAs of cytokines, prior to applying the above methods. More specifically, adipogenesis inhibitory factor can be produced in other animal cells, followed by screening.

DNA coding for adipogenesis inhibitory factor can be obtained from the thus obtained transformant of interest in accordance with known techniques [c.f., Maniatis, T. et al. (1982), "Molecular Cloning - A Laboratory Manual", Cold Spring Harbor Laboratory, NY]. For example, plasmid DNA can be isolated from the cells, followed by isolation of the cDNA from the plasmid DNA.

The sequence of the DNA obtained in this manner can be determined, for example, by the Maxam - Gilbert chemical modification method [Maxam, A.M. and Gilbert, W. (1980), "Methods in Enzymology", 65, 499-559], or the dideoxynucleotide chain termination method using M13 phage [Messing, J. and Vieira, J. (1982), Gene, 19, 269-276].

The resulting fragment containing the gene coding for adipogenesis inhibitory factor can then be cloned once more into suitable vector DNA, so as to be able to transform the host cells of other prokaryotes or eukaryotes. Moreover, by introducing a suitable promoter and sequence involved with gene expression into these vectors, the gene can be expressed in the respective host cells.

Examples of prokaryotic host cells include, for example, Escherichia coli and Bacillus subtilis. In order to express the gene of interest in these host cells, the host cells can be transformed with a replicon derived from a species compatible with the host or, in other words, with a plasmid vector containing a replication origin point and regulator sequences. In addition, it is desirable that the vector have a sequence able to provide transformed cells with a selectable expression character (phenotype).

For example, the K12 strain of E. coli is frequently used for the host, while pBR322 and pUC plasmids are commonly used for the vector. However, the present invention is not restricted to the use of these, and any of the various known types of bacterial strains and vectors can be used. Examples of promoters include the tryptophan (trp), lactose (lac), tryptophan-lactose (tac), lipoprotein (lpp), bacteriophage-originating lambda ($\lambda$) $P_L$ and polypeptide chain extending factor Tu (tufB) promoter in Escherichia coli. Any of these promoters can be used in production of the adipogenesis inhibitory factor of the present invention.

If Bacillus subtilis is used for the host cell, then the preferred strain is 207-25, and pTUB228 [Ohmura. K. et al. (1984), J. Biochem., 95, 87-93] and so forth used for the vector. However, the present invention is not limited to these. The regulator sequence of the $\alpha$-amylase gene of Bacillus subtilis is frequently used for the promoter. Secretion outside the bacteria can further be achieved by using a DNA sequence coding for the signal peptide sequence of $\alpha$-amylase, as necessary.

Eukaryotic host cells include vertebrate, insect and yeast cells. While COS cells, derived from monkey cells [Gluzman, Y. (1981), Cell, 23, 175-182], and the dihydrofolate reductase deficient strain of Chinese hamster ovary cells (CHO) [Urlaub, G. and Chasin, L.A. (1980), Proc. Natl. Acad. Sci. USA, 77, 4216-4220], etc. are frequently used where it is desired to use vertebrate cells, the present invention is not limited thereto.

Vectors having a promoter located upstream from the gene to be expressed, an RNA splicing site, a polyadenylation site or a transcription termination sequence, etc. can be used for the expression vector of the vertebrate cell. Such vectors may also have a replication origin, as necessary. The expression vector is exemplified by pSV2dhfr, which has an SV40 early promoter [Subramani, S. et al. (1981), Mol. Cell. Biol., 1, 854-864]. However, the present invention is not limited to this expression vector.

A yeast is typically used as a eukaryotic host. Particularly preferred are Saccharomyces, such as Saccharomyces cerevisiae. Expression vectors for the eukaryotic microorganism, such as yeast, preferably use, for example, the alcohol dehydrogenase gene promoter [Bennetzen, J.L. and Hall, B.D. (1982), J. Biol. Chem., 257, 3018-3025] or the acid phosphatase gene promoter [Miyanohara, A. et al. (1983), Proc. Natl. Acad. Sci. USA, 80, 1-5].

COS cells are a suitable example of host cells. A suitable vector for COS cells is autonomously replicable in COS cells, and has an SV40 replication origin, a transcription promoter, a transcription termination signal and an RNA splicing site. Transformation of the COS cells by the vector can be achieved by using the DEAE-dextran method [Luthman, H. and Magnusson, G. (1983), Nucleic Acids Res., 11, 1295-1308], the calcium phosphate-DNA coprecipitation method [Graham, F.L. and van der Ed, A.J. (1973), Virology, 52, 456-457], or the electroporation method [Neumann, E. et al. (1982), EMBO J., 1, 841-845].

If CHO cells are used, transformed cells that stably produce adipogenesis inhibitory factor can be obtained by co-transfecting the expression vector together with selectable vector capable of expressing a neo gene functioning as a G418 resistance marker, such as pRSVneo [Sambrook, J. et al. (1989), "Molecular Cloning - A Laboratory Manual", Cold Spring Harbor Laboratory, NY] or pSV2-neo [Southern, P.J. and Berg. P (1982), J. Mol. Appl. Genet., 1, 327-341], followed by selection of G418 resistant colonies.

The desired transformant thus obtained can be cultured by conventional procedures, and adipogenesis inhibitory factor can be produced either inside or outside the cells. The culture medium used can suitably be selected from the various types commonly used, according to the host cells employed. Examples of media for COS cells include RPMI-1640 medium and Dulbecco-modified Eagle's minimal essential medium (DMEM) to which a serum component, such as foetal bovine serum (FBS), has been added as necessary.

Thus, the adipogenesis inhibitory factor produced either inside or outside the cells of the transformant can be isolated and purified by various types of known isolation procedures, taking advantage of the physical properties and chemical properties, etc. of adipogenesis inhibitory factor. Practical embodiments of the methods include treatment by ordinary protein precipitants, ultrafiltration, various types of liquid chromatography such as molecular sieve chromatography (gel filtration), adsorption chromatography, ion exchange chromatography, affinity chromatography and high performance liquid chromatography (HPLC), dialysis or combinations of these methods.

According to the above methods, the desired adipogenesis inhibitory factor can easily be manufactured on an industrial scale, both in high yield and high purity. The various physical properties of the recombinant adipogenesis inhibitory factor of the present invention obtained in this manner are described in detail in the following Examples.

Adipogenesis inhibitory factor is useful in various fields (described above) by virtue of its biological activities.

Protein exhibiting adipogenesis inhibitory activity consists of 178 amino acids having Pro (amino acid number 1 of the amino acid sequence indicated in sequence number 2 of the sequence listing) as its N terminal, or in other words, that protein considered to be mature adipogenesis inhibitory factors. This protein has an apparent molecular weight of approximately 23,000 in SDS-polyacrylamide gel electrophoresis under reducing conditions.

In addition, DNA encoding the mature adipogenesis inhibitory factor also serves as the DNA of the present invention. More preferably, the DNA is the DNA consisting of nucleotide numbers 81 to 614 of the nucleotide sequence indicated in sequence number 1 of the sequence listing.

In general, the genes of eukaryotes are considered to demonstrate polymorphism, such as is known in the case of the interferon gene [c.f., Nishi, T. et al. (1985), J. Biochem., 97, 153-159]. Where one or more nucleotides are substituted as a result of this polymorphism, it is occasionally possible for all of the various amino acids to remain the same, despite a change in the nucleotide sequence.

Proteins corresponding to the mature adipogenesis inhibitory factor (amino acid numbers +1 to +178 of the amino acid sequence indicated in sequence number 2), and wherein one or more amino acid residues are deleted, at least at one site, or wherein are of the amino acid residues is substituted at one of the sites of said protein, may also possess adipogenesis inhibitory factor activity. These proteins are referred to herein as adipogenesis inhibitory factor equivalents.

For example, it is known that the cysteine codon of the interleukin 2 (IL-2) gene can be changed to a serine codon, and that the resulting protein keeps its IL-2 activity [Wang, A. et al. (1984), Science, 224, 1431-1433]. For this reason, all proteins, of either natural or synthetic origin, and which still possess adipogenesis inhibitory activity, are included in the present invention.

In addition, DNA encoding these proteins are also included in the present invention.

The various types of DNA of the present invention can be chemically synthesised by conventional methods, such as the phosphite-triester method [Hunkapiller, M. et al. (1984), Nature, 310, 105-111], based on the sequence data for the adipogenesis inhibitory factor.

Various codons for the desired amino acids are known. Suitable codons may be selected arbitrarily, or may be determined in accordance with commonly used methods [viz., Grantham, R. et al. (1981), Nucleic Acids Res., 9, r43-r74] taking into account, for example, frequency of codon usage in the host concerned. Partial alteration of the codons of these nucleotide sequences can be performed by conventional procedures, such as site specific mutagenesis [Mark, D.F. et al. (1984), Proc. Natl. Acad. Sci. USA, 81, 5662-5666], using a synthetic oligonucleotide primer coding for the desired mutation.

The adipogenesis inhibitory factor of the present invention may be administered either alone, or together with other therapeutic drugs, for the treatment of aplastic anaemia, leukopaenia caused by toxins or radiation, infections caused by viruses, bacteria and parasites, cytopaenia following bone marrow transplantation, refractory pancytopaenia and other incidental immunological disorders. Adipogenesis inhibitory factor may also be used either alone or in combination with other therapeutic drugs in the prevention and treatment of pathological obesity.

The cytopaenia ameliorant and/or anti-obesity compositions of the present invention comprise a mixture of a medically acceptable carrier and a therapeutically effective amount of adipogenesis inhibitory factor. The composition may be administered in various forms, for example, tablets, capsules, granules, powders or syrup for oral administration; and injections, intravenous drip or suppositories for parenteral administration.

Where the route of administration is by injection or intravenous drip, the therapeutic composition of the present invention is in the form of a non-pyrogenic, parenterally acceptable, aqueous solution. Preparation of a parenterally acceptable protein solution, taking into consideration pH, isotonicity and stability, is within the technological scope of those skilled in the art.

Dosage and form of administration in the treatment of the above conditions can be determined by a physician, taking into consideration factors having an effect on the action of the preparation, such as the condition of the patient, body weight, sex, age, diet, importance of other infections, administration time and other clinically significant factors. Normally, for oral administration, about 0.01 to 1,000 mg may be administered per day to an adult, either all at once or in subdivided doses. For non-oral administration, about 0.01 to 100 mg may be injected all at once either subcutaneously, intramuscularly or intravenously.

The cytopaenia ameliorant composition of the present invention can be used in combination with other haematopoietic factors, such as IL-1 - IL-10, LIF, stem cell factor, GM-CSF, G-CSF, M-CSF, Meg-CSF, TNF, IFN and erythropoietin. While other haematopoietic factors can be admixed as one of the components of the cytopaenia ameliorant composition of the present invention, they can also be administered to the patient as a separate composition. Additionally, use of the compositions of the present invention can enhance the activity or effect of treatment of other haematopoietic factors.

The anti-obesity composition of the present invention can be used in combination with other suitable anti-obesity drugs, such as anorectics, intestinal absorption inhibitors, digestive enzyme inhibitors, metabolic acceleration hormone preparations, fat synthesis inhibitors and insulin secretion inhibitors. Moreover, it can be used in combination with alimentary therapy and exercise therapy. In such cases, the use of the anti-obesity composition can promote the activity or effect of treatment of other anti-obesity drugs, as well as the effect of treatment of other anti-obesity therapies.

Because the cytopaenia ameliorant or anti-obesity preparations of the present invention are of biological origin, or are derived from genetic recombinants, they have low levels of toxicity. Proteins of the present invention were administered, per os. to adult male ddy strain mice (having body weights of about 20 g) at levels of 500 mg/kg or less for 5 days. None of the proteins of the present invention demonstrated toxicity.

In the Examples which follow, reference is had to the accompanying drawings, in which:

Fig. 1 is the restriction enzyme map of the cDNA coding for adipogenesis inhibitory factor, in which ORF represents the open reading frame;

Fig. 2 shows, schematically, the construction of a plasmid that expresses adipogenesis inhibitory factor according to accompanying Example 6; and

Fig. 3 shows an SDS-PAGE chromatogram and a Western blot using anti-peptide antibody under reducing conditions for total protein contained in the culture supernatant from COS-1 cells transfected with pcDL-SR $\alpha$-296 and pSR $\alpha$-20-2.

The present invention will be described below more specifically by way of non-limiting Examples, a Reference Example and a Preparation Example. The methods employed for measuring the various activities of adipogenesis inhibitory factor are described in the accompanying Reference Example.

Reference Example

Adipogenesis Inhibition

Adipogenesis inhibitory activity was assayed as follows. The cells used in the measurement were the mouse embryonic fibroblast cell line 3T3-L1 [Green, H. and Kehinde, O. (1974), Cell, 1, 113-116], purchased from the American Cell Type Culture Collection (ATCC). Cell culture was performed using medium A (DMEM 4.5 g/l of glucose, manufactured by Gibco) containing 10% immobilised FBS (manufactured by Hyclone) and 10 mM HEPES (pH 7.2, manufactured by Sigma)), at 37°C in the presence of a humidified gaseous mixture of 10% $CO_2$ and 90% air. Induction of differentiation of cells into adipocytes was performed in accordance with the method described by Rubin [Rubin, C.S. et al. (1978), J. Biol. Chem., 253, 7570-7578].

Assay for Suppressive Activity on Differentiation of 3T3-L1 Cells into Adipocvtes

3T3-L1 cells were cultured by suspending the cells in medium A (defined above) at a density of 1.0 x $10^4$ cells/ml. 0.5 ml of the cell suspension was pipetted into each well of a 48-well multicluster dish (Costar). After 3 days, the cells became confluent. The medium was then replaced with fresh medium A and, after culturing for an additional 2 days, the medium was replaced with medium B [DMEM (4.5 g/l of glucose) containing 10 mM HEPES (pH 7.2), 3% non-activated FBS, 5 µg/ml of bovine insulin (Sigma), 8 µg/ml of d-biotin (Sigma), 4 µg/ml of pantothenic acid (Sigma),

1.0 μM dexamethasone (Sigma) and 0.5 mM isobutylmethylxanthine (Aldrich)], to induce adipogenic differentiation. The sample of adipogenesis inhibitory factor was added at this time. Subsequently, the medium was replaced with fresh medium B every 2 days, and sample re-added. On the 4th-7th day after medium B and sample were first added, the medium was replaced with medium C [DMEM (containing 4.5 g/l of glucose) containing 51; immobilised FBS, 10 mM HEPES (pH 7.2) and 100 ng/ml of bovine insulin], to sustain adipocytes.

After culturing for 2 days in medium C, the cells were fixed with 5% formaldehyde. The fat droplets that had accumulated within the cells and cell nuclei were stained with oil red O and haematoxylin, respectively. Ater micrographs had been taken, the number of cells in which red stained fat droplets had accumulated, determined from the photographs, along with the number of nucleated cells stained with haematoxylin, were counted. The rate of adipogenic differentiation was then calculated using the formula shown below.

$$\text{Adipogenic Differentiation Rate (\%)} = 100 \times \frac{\text{No. of Cells Containing Fat Droplets}}{\text{No. of Nucleated Cells}}$$

Cell fixation, oil red O staining and haematoxylin staining were performed in accordance with the experimental manual "Mitomo, Y. and Takayama S., "RINSHOKENSAKOZA (Clinical Testing Seminar)", Vol. 12, "Pathology" (1982), Ishiyaku Publishing".

Assay of LPL Suppressing Activity

The assay was performed substantially in accordance with the method described by Beutler [Beutler, B.A, et al. (1985), J. Immunol., 135, 3972-3977]. Fat cell transformed 3T3-L1 adipocytes were prepared by the method described above (Assay for Suppressive Activity on Differentiation of 3T3-L1 Cells into Adipocytes), with the exception that no sample was added when differentiation into adipocytes was induced. The medium was then replaced with fresh medium C, and sample added. After culturing for 18 hours, the medium was removed and the cells were washed twice with PBS(-) (phosphate buffered saline, Nissui Seiyaku). Next, 300 μl of medium D [DMEM (4.5 g/l glucose) containing 5% immobilised FBS, 10 mM HEPES (pH 7.2), 100 ng/ml of bovine insulin and 10 U/ml sodium heparin (Nobo Industries)] was added to each well. After culturing for 1 hour, 100 μl of the culture supernatant was collected and used for measurement of LPL activity. Measurement of LPL suppressing activity was performed three times for each sample, and the average of the three measured values was calculated.

Measurement of LPL activity was performed in accordance with the method of Nilsson-Ehle and Schotz [Nilsson-Bhle, P. and Schotz, M.C. (1976), J. Lipid Res., 17, 536-541]. 100 1 of culture supernatant, prepared as described above, was mixed with an equal volume of substrate solution {13 μM glycerol-tri-[9,10(n)- $^3$H]oleic acid (51.8 KBeq/μmol, Amersham, and prepared as described below), 1.3 mg/ml L-α distearoyl phosphatidylcholine (Sigma), 20 mg/ml bovine serum albumin (Sigma), 135 mM Tris- hydrochloride (Tris-HCl, pH 8.1, Sigma), 16.5% (v/v) glycerol and 16.5% (v/v) immobilised FBS}, and allowed to react at 37°C for 120 minutes. The reaction was stopped by addition of 1.05 ml of 0.1 M potassium carbonate-boric acid buffer (pH 10.5) and 3.25 ml of methanol : chloroform : heptane = 141 : 125 : 100 (v/v). After vigorous stirring, the reaction mixture was centrifuged at 3,000 x g for 15 minutes. The $^3$H count of the water-methanol layer was then measured using a liquid scintillation counter. 1 unit of LPL activity was defined as the amount of activity producing 1 μmol of fatty acid in 1 minute. Glycerol-tri[9,10(n)-$^3$H]oleic acid (used in the substrate solution) was prepared by diluting glycerol-tri[9,10(n)-$^3$H]oleic acid (Amersham, 37.0 GBeq/mol) with triolein (Sigma), followed by purification using silica gel column chromatography.

Example 1:

Isolation of Poly(A)$^+$RNA from KM-102 Cells

KM-102 cells were cultured in 36 plastic culture dishes (15 cm in diameter) with Iscove's modified minimal essential medium (manufactured by Boehringer-Mannheim) containing 10% FBS. After the cells had been grown to confluence, phorbol myristate acetate (PMA) and the calcium ionophore A23187 were added to concentrations of 10ng/ml and 0.2 μM, respectively. Culturing was then continued, and the cells from 12 dishes at a time were lysed with guanidine-thiocyanate solution [4 M guanidine thiocyanate, 1% sarcosil, 20 mM ethylenediaminetetraacetic acid (EDTA), 25 mM sodium citrate (pH 7.0), 100 mM 2-mercaptoethanol and 0.1% antifoam A] after 3, 6 and 14 hours, respectively, and the solution recovered.

Isolation of poly(A)$^+$-RNA was essentially performed as described in "Molecular Cloning - A Laboratory Manual" [Maniatis, T. et al. (1982), pp. 196-198]. More specifically, the isolation was performed as described below.

The recovered lysed cell solutions were repeatedly taken up and discharged using 10 ml syringes equipped with 21G needles. Polyalomar centrifuge tubes compatible with the RPS-40T rotor bucket manufactured by Hitachi Koki each had 3 ml of 5.7 M CsCl-0.1 M EDTA (pH 7.5) added thereto, and the lysed cell solutions were overlaid on the previously added solution until the tubes had been filled. After these solutions had been centrifuged for 18 hours at 20°C and at 30,000 rpm, the resulting pellets were dissolved in 400 µl of distilled water, followed by precipitation with ethanol. The resulting pellets were then redissolved in 400 µl of distilled water, and an equal volume of chloroform : 1-butanol (4 : 1) was added thereto, followed by stirring. The aqueous layer was then recovered by centrifugal separation. After the aqueous layer had been precipitated with ethanol, the precipitated pellets were dissolved in 600 µl of distilled water to obtain total RNA. About 4.5 mg each of total RNA was obtained from each sample after 3, 6 and 14 hours of stimulation with PMA-A23187.

600 µg each of the three batches of total RNA obtained above were mixed together, and the mixture was subjected to oligo(dT) cellulose column chromatography to purify poly(A)$^+$-RNA. More specifically, after dissolution of total RNA in an adsorption buffer [0.5 M NaCl, 20 mM Tris-HCl (pH 7.5), 1 mM EDTA and 0.1% sodium dodecyl sulphate (SDS)] and heating at 65°C for 5 minutes, the resulting solution was applied to an oligo(dT) cellulose column (Pharmacia, Type 7) filled with the same solution. Poly(A)$^+$-RNA was recovered by elution with an eluent [10 mM Tris-HCl (pH 7.5), 1 mM EDTA and 0.05% SDS] to obtain 100 µg of poly(A)$^+$RNA.

Example 2:

Preparation of a cDNA Library

Preparation of a cDNA library was performed by the Okayama-Berg method. More specifically, 5 µg of poly (A)$^+$-RNA and 24 units of reverse transcriptase were allowed to react in a reaction mixture {50 mM Tris-HCl (pH 8.3), 8 mM $MgCl_2$, 30 mM KCl, 0.3 mM dithiothreitol (DTT), 2 mM dATP, 2 mM dGTP, 2 mM dCTP, 2 mM dTTP, 10 µ Ci [$\alpha$-$^{32}$P]dCTP and 1.4 µg of vector primer DNA [3'-oligo(dT)-tailed pcDV-1, Pharmacial} at 42°C for 60 minutes.

After the reaction had been stopped by addition of 2 µl of 0.25 M EDTA and 1 µl of 10% SDS, protein was removed by treatment with 20 µl of phenol-chloroform (1 : 1). 20 µl of 4 M ammonium acetate and 80 µ of ethanol were added to the aqueous layer which had been collected by centrifugation, followed by cooling at - 70°C for 15 minutes. The precipitate was then collected by centrifugation and washed with 75% ethanol, followed by drying under reduced pressure.

The dried precipitate was then dissolved in 15 µl of a terminal transferase reaction mixture [140 mM potassium cacodylate, 30 mM Tris-HCl (pH 6.8), 1 mM $CoCl_2$, 0.5 mM DTT, 0.2 µg of polyA and 100 mM dCTP]. The reaction mixture was kept at 37°C for 3 minutes, after which 18 units of terminal deoxynucleotidyl transferase were added and allowed to react for 5 minutes. Protein was then removed by treatment with phenol-chloroform, after the reaction had been stopped by addition of 1 µl of 0.25 M EDTA and 0.5 µl of 10% SDS. After centrifugation of the reaction mixture, the aqueous layer was recovered, and 15 µl of 4 M ammonium acetate and 60 µl of ethanol were added thereto, followed by thorough mixing. After cooling of the mixture at -70°C for 15 minutes, the precipitate thus formed was collected by centrifugation.

This precipitate was then dissolved in 10 µl of restriction enzyme buffer [50 mM NaCl, 10 mM Tris-HCl (pH 7.5), 10 mM $MgCl_2$ and 1 mM DTT], 2.5 units of the restriction enzyme Hind III were added, and the precipitate was digested at 37°C for about 1 hour.

Proteins were then removed by treatment with phenol-chloroform and, aferwards, ethanol precipitation was performed. After the reaction mixture had been cooled at -70°C for 15 minutes, the precipitate was collected by centrifugation and dissolved in 10 µl of TE buffer [10 mM Tris-HCl (pH 7.5) and 1 mM EDTA] . 1 µl of this solution was added to 9 µl of a reaction mixture [10 mM Tris-HCl (pH 7.5), 1 mM EDTA and 100 mM NaCl] to which 10 ng of oligo (dG)-tailed linker-DNA [3'-oligo(dG)tailed pL-1 Hind3 linker, Pharmacia] was added, followed by heating at 65°C for 5 minutes. The reaction mixture was left to stand at 42°C for 30 minutes. The reaction mixture was then cooled in ice water, after which 10 µl of 10x ligase buffer [10 mM ATP, 660 mM Tris-HCl (pH 7.5), 66 mM $MgCl_2$ and 100 mM DTTI, 78 µl of distilled water and 8 units of T4 DNA ligase were added, and the mixture was allowed to stand at 12°C overnight.

10 µl of 1 M KCl, 1 unit of ribonuclease H, 33 units of DNA polymerase I, 4 units of T4 DNA ligase, 0.5 µl of a nucleotide solution (20 mM dATP, 20 mM dGTP, 20 mM dCTP and 20 mM dTTP) and 0.1 µl of bovine serum albumin (50 µg/µl) were then added, and the mixture was kept at 12°C for 1 hour, then at 25°C for 1 hour. After the reaction mixture had been diluted five times with distilled water, Escherichia coli DH5 $\alpha$ was immediately transformed in accordance with the method described by Hanahan [Hanahan, D. (1983), J. Mol. Biol., 166, 557-580] to provide a KM-102 cell cDNA library.

Example 3

Preparation of Oligonucleotide Probe

The 15-mer 5'-TAAATAAATAAATAA-3', designated ATT-3, was chemically synthesised based on the AUUUA sequence conserved in the 3'-untranslated region of cytokine mRNA. Synthesis was performed using a 380B Automatic DNA Synthesiser (Applied Biosystems) according to the procedures set out in the manual. This method is based on the principle described by Caruthers et al. [Metteucci, M.D. and Caruthers, M.H. (1981), J. Am. Chem. Soc., 103, 3185-3191], and is known as the phosphoamidite method. After the 15 nucleotides had been synthesised, they were deprotected by cleaving from the support resin, and the resulting mixture was freeze-dried to provide an oligonucleotide powder. The powder was then dissolved in distilled water and stored, frozen at -20°C, until required.

Example 4

Screening of cDNA the Library

6,500 strains of recombinant DNAs from the cDNA library prepared in Example 3 were fixed on a nitrocellulose filter by the method of Grunstein, M. and Hogness, D.S. [Grunstein, M. and Hogness, D.S. (1975), Proc. Natl. Acad. Sci. USA, 72, 3961-3965]. The 5'-terminal of the probe (ATT-3) was labelled with $^{32}$ P by conventional procedures (see "Molecular Cloning - A Laboratory Manual", supra). after which, colony hybridisation was performed . Pre-hybridisation was performed at 37°C for 3 hours in a solution containing 6 X SSC (1 X SSC = 150 mM NaCl and 15 mM trisodium citrate), 1 X Denhardt's solution, 0.25% SDS, 0.05% sodium pyrophosphate and 100 μg/ml of denatured salmon sperm DNA. Hybridisation was then performed at 31°C overnight in a reaction mixture of 6 X SSC containing the $^{32}$P radio-labelled probe (ATT-3), 1 X Denhardt's solution, 17 μg/ml of yeast tRNA and 0.05% sodium pyrophosphate. After completion of the reaction, the nitrocellulose filter was washed with 6 X SSC solution containing 0.05% sodium pyrophosphate at room temperature for 2 hours, and then subjected to autoradiography. As a result, 33 positive clones were obtained. Following isolation of plasmid DNA from these clones by the conventional procedure, several clones were selected at random, and a partial nucleotide sequence of the resulting cDNA was then determined by the dideoxy method. Homology with nucleotide sequences registered in the EMBL or GenBank data bases was then checked for, using a personal computer. It was, thus, determined that the region to which the ATT-3 probe hybridised was homologous with members of the Alu repetitive sequence [Schmid, C.W. and Jelinek, W.R. (1982), Science, 216, 1065-1070].

When a DNA fragment containing the Alu repetitive sequence prepared from human genome DNA was labelled with $^{32}$P and used as a probe to perform colony hybridisation of the above-mentioned 33 clones, it was established that 12 of the clones possessed the Alu repetitive sequences. The length of the cDNA insert was determined for the remaining 21 clones, and it was found that the inserts contained from 50 to 3,600 bases. After mapping of the cDNA of the 21 clones with restriction enzymes, the nucleotide sequences of the cDNA were partially determined. The above-mentioned data bases were searched for homologous sequences, and clones having new sequences not registered in those data bases were selected.

Since these plasmids contain an SV40 early promoter and a replication origin, they are suited for expression of cDNA in COS-1 cells. Therefore, plasmid DNA for the clones selected as mentioned above was introduced into COS-1 cells. Transfection of the COS-1 cells was performed by electroporation using the Shimadzu GTE-1 gene implantation unit.

More specifically, COS-1 cells were grown in a flask to a semi-confluent state, the cells were collected by treatment with trypsin-EDTA and washed twice with PBS(-) buffer (Nissui Seiyaku). Next, the cells were suspended in a PBS(-) buffer at 6 x $10^7$ cells/ml. On the other hand, each of the plasmid DNAs prepared by the caesium chloride method were adjusted to 200 μg/ml with PBS(-) buffer. 20 μl each of the above-mentioned cell suspension and DNA solution were mixed together and placed in a chamber having an electrode separation of 2 mm. 30 μsec pulses of 600V were then applied twice at 1 second intervals. After cooling the chamber at 4°C for about 5 minutes, the cell-DNA mixture in the chamber was added to 10 ml of DMEM containing 10% FBS. The resulting mixture was then transferred to a dish and cultured in the presence of 5% $CO_2$ at 37°C overnight. The culture supernatant was then removed, the cells were washed with serum-free medium (DMEM), and 10 ml of DMEM was added, followed by culturing for 3 days. The supernatant was recovered from the cell culture obtained, and assayed for suppressive activity on the differentiation of 3T3-L1 Cells into adipocytes, as described in the reference Example.

Adipocyte inhibitory activity was detected solely for the culture supernatant of COS-1 cells transfected with the plasmid (designated pcD-20-2) of the clone which we had given the number 20-2. Adipogenesis transformation inhibitory activity data are shown in Table 1.

Table 1

| Additive | Adipogenesis Differentiation Rate (%) |
|---|---|
| Serum-free DMEM (blank) | 72 |
| Culture supernatant of COS-1 cells transfected with pUC-CAT (4% addition) | 41 |
| Culture supernatant of COS-1 cells transfected with pcD-20-2 (4% addition) | 13 |

pUC-CAT is a plasmid obtained by inserting a chloramphenicol acetyl transferase gene into a pUC vector, and can be used as a negative control, since it does not have a promoter which works in mammalian cells. Transfection was performed in the same manner as for pcD-20-2. As shown in Table 1, inhibitory activity on the differentiation of 3T3-L1 cells into adipocytes could be detected in the culture supernatant of COS-1 cells transfected with pcD-20-2. In addition, in those 3T3-L1 cells induced to differentiate into adipocytes (Table 2), LPL activity was strongly suppressed. The values in the Table represent a relative value (%) on the basis that 100% LPL activity (blank) is taken as that which occurs when only serum-free DMEM is added.

Table 2

| Additive | Relative Value of LPL Activity (%) |
|---|---|
| Serum-free DMEM (blank) | 100 |
| Culture supernatant of COS-1 cells transfected with pUC-CAT (4% addition) | 95 |
| Culture supernatant of COS-1 cells transfected with pcD-20-2 (4% addition) | 38 |

A restriction enzyme map of the cDNA insert of pcD-20-2 was prepared (Fig. 1). The entire nucleotide sequence of the cDNA was then determined by the dideoxy method, and the nucleotide sequence indicated in sequence number 1 (SEQ ID NO: 1) of the sequence listing was obtained. It was found that the cDNA insert of pcD-20-2 consists of 1065 bases with a poly(A) tail (41 A residues). It was also found that the cDNA has an open reading frame (ORF) consisting of 199 amino acids starting with methionine. When the resulting nucleotide sequence was compared with the sequences in the EMBL and GenBank data bases, no homology with any known sequence was found. The amino acid sequence encoded by the ORF was also compared with the NBRF and SWISS prot data bases, and no homology with any known sequence was observed. It was established, therefore, that the ORF of the cDNA insert of pcD-20-2 encodes a novel polypeptide. The amino acid sequence encoded by the ORF is indicated in sequence number 2 (SEQ ID NO: 2) of the sequence listing.

A region containing strongly hydrophobic amino acids (a signal peptide), observed in various secretory proteins, is present in the N-terminal region of the amino acid sequence, starting with methionine. This suggests that the protein is a secretory protein.

Example 5

Peptide Synthesis and Preparation of Anti-peptide Antiserum

The peptide corresponding to the amino acid sequence 27 - 41 from the methionine at the N-terminal ($NH_2$-Gly-Pro-Pro-Arg-Val-Ser-Pro-Asp-Pro-Arg-Ala-Glu-Leu-Asp-Ser-COOH) was synthesised in order to confirm that the novel peptide coded for by the ORF of the cDNA insert of pcD-20-2 is a secretory protein, and also to detect adipogenesis inhibitory factor during purification of the factor, described later. The peptide thus synthesised was then used as an antigen to immunise a rabbit and to prepare antiserum.

In more detail, peptide synthesis was performed according to the tBOC-amino acid solid phase method [Merrifield, R.B. (1963), J. Am. Chem. Soc., 85, 2149-2154] following a standard automatic synthesis program using a peptide synthesiser 430A manufactured by Applied Biosystems. After deprotection and removal of resin, the peptide was purified on an anion exchange column. Confirmation of the main peak pattern was performed by HPLC using a reverse phase column. Following concentration of the peptide by freeze drying, the peaks were separated and collected by HPLC using a reverse phase column. After concentration of the protein had again been performed by freeze drying, HPLC analysis and amino acid composition analysis were carried out. amino acid composition was analysed using a Pico Tag System, an automated amino acid analyser manufactured by Waters.

Keyhole Limpet Hemocyanin (KLH), serving as the carrier protein, was coupled to the resulting peptide by the glutaraldehyde (GAD) method [Konopka, J.B. et al. (1984), J. Virol., 51, 223-232], and used as the antigen for rabbit immunisation. The antigen was suspended homogeneously in Freund's complete adjuvant (FCA), and a rabbit was immunised by administration of the suspension subdermally to the back. Whole blood was collected after administering the suspension three times at about two week intervals. Measurement of the antibody titre in the rabbit serum was performed by enzyme-linked immunosorbent assay (ELISA). An IgG fraction was prepared from the resulting antiserum using a Protein A-Sepharose 6MB column manufactured by Pharmacia. This was then used as a primary antibody.

Example 6

Production and Purification of Adipogenesis Inhibitory Factor in COS-1 Cells

i) Construction of a High Expression Vector and Expression in COS-1 Cells

Following digestion of pcD-20-2 with Bam H1, the 1240 bp fragment containing the cDNA insert was isolated and purified. Meanwhile, following digestion of the high expression vector pcDL-SR α296 [Takebe, Y. et al. (1988), Mol. Cell. Biol., 8, 466-472], also with BamH1, the 3.4 kb fragment containing the SR α promoter was prepared, and the cDNA Bam H1 fragment was ligated thereto by a reaction using T4 DNA ligase. The resulting DNA was then used to transform Escherichia coli DH5 α, and an analysis of the plasmid in the resulting transformant was performed. A strain in which the direction of cDNA transcription is the same as the direction of the SR α promoter was selected, and this plasmid was named pSR α-20-2 (Fig. 2).

The SR promoter comprises an SV40 early promoter and the R-U5 sequence of the long terminal repeat (LTR) of HTLV-1, and demonstrates a promoter activity 10 to 100 times greater than that of SV40 early promoter alone.

COS-1 cells were then transfected with the resulting pSR α-20-2 plasmid to confirm, using the antibody obtained in Example 5, that adipogenesis inhibitory factor is secreted into the culture supernatant. Transfection of COS-1 cells was performed either by the electroporation method or by the DEAF-dextran method [Luthman, H. and Magnusson G. (1983), Nucleic Acid Res., 11, 1295-1308]. 160 µl of serum-free culture supernatant obtained from each of: COS-1 cells transfected with negative control plasmid; COS-1 cells transfected with pcDL-SR α296 containing no cDNA; and COS-1 cells transfected with pSR α-20-2 and expressing adipogenesis inhibitory factor, were treated with trichloroacetic acid (TCA) to precipitate protein, and the precipitate was then obtained by centrifugal separation. After the precipitate had been washed using ice-cooled acetone and then air-dried, it was dissolved in a sample buffer containing 2-mercaptoethanol and subjected to SDS-polyacrylamide gel electrophoresis (SDS-PAGE) under reducing conditions using a 12.5% gel. Detection of the protein bands after electrophoresis was performed by silver staining using the Silbest Stain manufactured by Nacalai Tesque (panel at the left of Fig. 3). A single band having a specific pattern was detected at the position indicated by the arrow (molecular weight: approximately 23,000) in the culture supernatant of COS-1 cells transfected with pSR α-20-2.

In addition, Western blot analysis was performed using the primary antibody prepared in Example 5, after the protein bands obtained by polyacrylamide gel electrophoresis had been blotted onto a nitrocellulose membrane using a gel membrane blotter (manufactured by Marysol, KS-8441) in accordance with the method of Towbin et al. [Towbin, H. et al. (1979), Proc. Natl. Acad. Sci. USA, 76, 4350-4354]. The Western blotting procedure was performed using the Immun-Blot (GAR-HRP) Assay Kit manufactured by Bio-Rad, according to the instructions of the manufacturer (panel on the right of Fig. 3). Only the specific band detected by silver staining (molecular weight: approximately 23,000) reacted with the antipeptide antibody, thereby confirming that the protein encoded by the ORF of the cDNA insert of pcD-20-2 is a secretory protein.

A study was then conducted of the adipogenesis differentiation inhibitory activity of the culture supernatant of COS-1 cells transfected with pSR α-20-2. Activity data are shown in Table 3.

Table 3

| Additive | Adipogenic Differentiation Rate (%) |
|---|---|
| Serum-free DMEM (blank) | 85 |
| Culture supernatant of COS-1 cells transfected with pcDL-SR α296 (0.6% addition) | 84 |
| Culture supernatant of COS-1 cells transfected with pSR α-20-2 (0.6% addition) | 29 |

Although adipogenesis differentiation inhibitory activity is not observed in the culture supernatant of COS-1 cells transfected with pcDL-SR α296, (negative control containing no cDNA insert), remarkable activity was detected in the case of pSR α-20-2. In addition, LPL activity was also strongly suppressed in 3T3-L1 cells which had been induced to differentiate into adipocytes (Table 4). The inhibitory activity indicated in Table 3 and Table 4 was remarkably stronger than for when pcD-20-2 had been used (Table 1 and Table 2).

Table 4

| Additive | Relative Value of LPL Activity (%) |
|---|---|
| Serum-free DMEM (blank) | 100 |
| Culture supernatant of COS-1 cells transfected with pcDL-SR α296 (0.8% addition) | 117 |
| Culture supernatant of COS-1 cells transfected with pSR α-20-2 (0.8% addition) | 17 |

ii) Purification and N Terminal Amino Acid Sequence Analysis

7 L of serum-free culture supernatant, obtained by transfection of COS-1 cells with pSR α-20-2 as described above, was dialysed with a 20-fold volume of dialysis buffer [10 mM boric acid-NaOH (pH 9.0) and 13 mM KCl] at 4°C for 15 hours, after which weak cation exchange chromatography was performed using the FPLC manufactured by Pharmacia under the conditions indicated below:

Column: CM-Toyopearl Pack 650M (2.2 x 20 cm, manufactured by Tosoh)

Elution buffer solution :

| | |
|---|---|
| Solution A: | 10 mM boric acid-NaOH (pH 9.0), 13 mM KCl |
| Solution B: | Solution A containing 300 mM NaCl |
| Flow rate: | 3 ml/min. |
| Fraction volume: | 3 ml/tube |
| Gradient of the concentration: | Linear concentration gradient from 100% solution A to 100% solution B over the course of 50 minutes |

Western blotting was performed on each of the fractions obtained, using the antipeptide antibody prepared in Example 5 to identify the fraction containing adipogenesis inhibitory factor (No's. 35 - 45). The total amount of the peak fraction (No. 41), containing the largest amount of adipogenesis inhibitory factor eluted with 260 mM NaCl, was concentrated by TCA precipitation treatment and analysed by SDS-PAGE, using a 12.5% gel under reducing conditions. Following electrophoresis, the protein bands from the polyacrylamide gel were blotted onto a polyvinylidene difluoride (PVDF) membrane (Millipore, Immobilon™) using a gel membrane blotter (Marysol, KS-8441) in a transfer buffer [0.02% SDS, 20% methanol and 25 mM Tris-borate (pH 9.5)] at 4°C for 15 hours and with a current of 0.8 mA/cm$^2$ . The blotted membrane was washed for 5 minutes with 10 mM sodium borate buffer (pH 8.0) containing 25 mM NaCl and then with distilled water with shaking for 5 minutes, after which the membrane was air-dried. Next, the portion to which adipogenesis inhibitory factor had been transferred was excised from the membrane, and the sequence to 10 amino acid residues from the N terminal was determined using a gas phase protein sequencer (manufactured by Applied Biosystems, Model 475A).

The phenylthiohydantoin (PHT)-amino acids obtained in the respective reaction cycles were separated and identified by reverse phase HPLC (Applied Biosystems, Model 120A). The amino acid sequence determined by the above-mentioned procedure is as indicated below, and corresponds to amino acids 1 - 10 of SEQ ID NO: 2.

Pro-Gly-Pro-Pro-Pro-Gly-Pro-Pro-Arg-Val-

This indicates that human adipogenesis inhibitory factor is secreted outside the cell in a mature form, starting with a Pro residue, after biosynthesis of a precursor composed of 199 amino acids, and the loss of the signal peptide composed of 21 amino acids at the N terminal by severing.

Example 7

Biological Activity of Purified Adipogenesis Inhibitory Factor

Adipogenesis inhibitory factor was purified from serum-free culture supernatant, obtained by transfection of COS-1 cells with pSR α-20-2, in accordance with known methods, combining weak cation exchange chromatography, hydrophobic chromatography and gel filtration column chromatography and so forth, and the factor was detected in the form of a single band by SDS-PAGE analysis. Remarkable activity was detected as a result of examining the adipogenesis differentiation inhibitory activity and LPL suprressing activity using this purified adipogenesis inhibitory factor.

Thus, it was concluded that the adipogenesis inhibitory factor of the present invention results from a single protein that possesses adipogenesis inhibitory factor activity.

Example 8

Secretory Production of Adipogesis Transformation Inhibitory Factor in CHO Cells

CHO cells in logarithmic growth phase were co-transfected with pSR α-20-2 and pSRVneo, at a DNA ratio of 5 : 1, using the calcium phosphate-DNA co-precipitation method. Selection of transformed strains was performed by culturing with HamF12 medium (Nissui Seiyaku) containing 400 μg/ml of G418 (Gibco Oriental) and 10% FBS for 9 days. After 15 strains had been selected from the resulting resistant colonies, the colonies were transferred to a 24-well plate using a cloning ring, and culturing continued. Those cells that reached a confluent state were transferred to square flasks and subcultured, and then adipogenesis inhibitory factor production for the 15 strains was assayed, using the Western blotting method described above.

The clone that demonstrated the highest production of adipogenesis inhibitory factor was selected and then cultured by a limiting dilution technique, using a 96-well plate. Four clones derived from this single cell were selected. The 4 strains of cells were transferred to culture dishes and cultured to a confluent state, and the amount of adipogenesis inhibitory factor was then assayed using the Western blotting method for each culture supernatant. The amounts of secretory production of adipogenesis inhibitory factor were stable, and there was no great difference in amounts between the 4 strains. In addition, the effects of the culture supernatants obtained above on the LPL activity of 3T3-L1 cells were investigated, and LPL suppressing activity was detected in the culture supernatants of all 4 clones. Next, one of the 4 strains was cultured in a serum-free medium (CHO-1 complete media system, manufactured by Ventrex). After the strain had been cultured in the serum-free medium for 2 days, subculturing was performed by treatment with trypsin-EDTA, followed by culturing for a further 3 days. The culture supernatant was then recovered and examined using the Western blotting method, and it was confirmed that adipogenesis inhibitory factor was being stably produced.

Example 9

Inhibitory Action on Adipogenic Differentiation of Mouse Bone Marrow - Derived Preadipocyte Cell Line H-1/A

Mouse bone marrow derived preadipocyte cell line H-1/A [Nakamura, M. et al. (1985), Proc. Soc. Exp. Biol. Med., 179, 283-287] was suspended at a density of $2.0 \times 10^4$ cells/ml in medium E [Fischer's medium (Gibco) containing 10% immobilized FBS (Hyclone)]. This cell suspension was then pipetted on to a Celltight C-1 Plate 48F (Sumitomo Bakelite), at 0.5 ml per well, and cultured at 33°C in a humidified gaseous mixture of 5% $CO_2$ and 95% air. Three days later, the medium was replaced with medium F [medium E containing 1 μM hydrocortisone (Sigma)] to induce adipogenic differentiation. At the same time, culture supernatant from COS-1 cells transfected with pSR α-20-2 plasmid, culture supernatant from COS-1 cells transfected with pcDL-SR α296 or serum-free DMEM medium was added. The medium was replaced with fresh medium F, and COS-1 cell culture supernatant or DMEM medium were added afresh, every 4 days. The cells were fixed with 5% formaldehyde on the 26th day after addition of medium F, and the fat droplets which had accumulated in the cells and cell nuclei were then stained using oil red O and haematoxylin, respectively. The adipogenic differentiation rate was then calculated using the same method as that used for the measurement of inhibitory action on adipogenic differentiation of 3T3-L1 cells. As shown in Table 5, strongly inhibitory adipogenic differentiation action on H-1/A cells was detected in the culture supernatant of COS-1 cells transfected with pSR α-20-2. Adipogenesis inhibitory factor purified from the culture supernatant of COS-1 cells transfected with pSR α-20-2 also remarkably inhibited adipogenic differentiation of H-1/A cells.

Table 5

| Additive | Adipogenic Differentiation Rate (%) |
|---|---|
| Serum-free DMEM (8% addition) | 34 |
| Culture supernatant of COS-1 cells transfected with pcDL-SR α296 (8% addition) | 36 |
| Culture supernatant of COS-1 cells transfected with pSR α-20-2 (8% addition) | 5 |

Example 10

CSF Inducing Action on Bone Marrow - Derived Preadipocyte Cell Line H-1/A

H-1/A cells were suspended at a density of 1.0 x $10^4$ cells/ml in medium E and pipetted at 3 ml per well into a 6-well cluster dish (Cosaer). The cells were cultured at 33°C in a humidified gaseous mixture of 5% $CO_2$ and 95% air. After the cells had been cultured for 6 days, the medium was replaced with fresh medium E. At this time, culture supernatant from COS-1 cells transfected with pSR α-20-2 plasmid, culture supernatant from COS-1 cells transfected with pcDL-SR α296 plasmid, or serum-free DMEM medium was added, and culturing continued for a further 5 hours.

After culturing, the cells were washed three times with serum-free Fischer's medium, and 3 ml of fresh medium E was added to each well, and culturing was continued for a further 14 hours. Culture supernatant was then collected from each well. The thus obtained culture supernatant was filtered using a Millex GV filter having a pore size of 0.22 μm (Nippon Millipore Industries), and the filtrate was then used for a colony stimulating activity test. The colony stimulating activity test was performed according to the method of Kubota et al. [Kubota, K. et al. (1981), Cancer Res., 41, 3052-3057]. Femoral bone marrow cells from female C3H He/N mice (purchased from Japan Charles River) were suspended at a density of 1.0 x $10^5$ cells/ml in RPMI 1640 medium (Gibco) to which 0.3% bactoagar (Difco), 20% FBS and 10% H-1/A cell culture supernatant had been added. Portions of 1 ml of this suspension were each pipetted into 35 x 10 mm plastic dishes (Lux). After the cells had been cultured at 37°C in a humidified gaseous mixture of 5% $CO_2$ and 95% air for 7 days, the number of colonies formed in each dish was assessed.

The culture supernatant of H-1/A cells treated with medium E, to which the culture supernatant from COS-1 cells transfected with pSR α-20-2 had been added, demonstrated significantly higher colony stimulating activity ($p<0.01$) over the culture supernatant of H-1/A cells treated with medium E to which the culture supernatant from COS-1 cells transfected with pcDL-SR α296 or serum-free DMEM had been added. The results are summarised in Table 6.

Table 6

| Additive | Number of Colonies (mean±S.D., n=6) |
|---|---|
| Culture supernatant of H-1/A cells treated with medium E containing 2.5% serum-free DMEM | 46.7 ± 13.8 |
| Culture supernatant of H-1/A cells treated with medium E containing 2.5% culture supernatant of COS-1 cells transfected with pcDL-SR α296 | 44.5 ± 13.6 |
| Culture supernatant of H-1/A cells treated with medium E containing 2.5% culture supernatant of COS-1 cells transfected with pSR α-20-2 | 72.0 ± 14.3 |

5.0 x $10^6$ H-1/A cells were suspended in 50 ml of medium E and cultured in an Accel flask (Costar) for 5 days. Next, the medium was replaced with fresh medium E, and culture supernatant from COS-1 cells transfected with pSR α-20-2, culture supernatant from COS-1 cells transfected with pcDL-SR α296, or serum-free DMEM was added, simultaneously. After the mixture had been cultured for 18 hours, total RNA was isolated from the cells in the same manner as in Example 1. The amount of M-CSF mRNA was analysed by the Northern hybridisation method [Thomas, P.S. (1980), Proc. Natl. Acad. Sci. USA, 77, 5201-5205] using 20 μg each of total RNA. Mouse M-CSF cDNA prepared by polymerase chain reaction [Ladner, M.B. et al. (1988), Proc. Natl. Acad. Sci. USA, 85, 6706-6710] was $^{32}$P-labelled using the Multiprime DNA Labelling System (Amersham) and used as the probe. The amount of hybridised $^{32}$P-labelled probe was determined using an image analyser (Fuji Photo Film). The $^{32}$P-labelled probe was hybridised to RNA which had sizes of about 2.5 kb and about 4.5 kb. When the total amounts of probe hybridised to these sizes of RNA were compared, the amount of M-CSF mRNA per unit total RNA was found to have increased in H-1/A cells treated with medium E to which 5% culture supernatant from COS-1 cells transfected with pSR α-20-2 had been added, when compared with H-1/A cells treated with medium E to which had been added 5% culture supernatant from COS-1 cells transfected with pcDL-SR α296 (Table 7).

Table 7

| Additive | Relative Value of Amount of M-CSF mRNA (%) |
|---|---|
| Culture supernatant of COS-1 cells transfected with pcDL-SR α296 (5% addition) | 100 |
| Culture supernatant of COS-1 cells transfected with pSR α-2C-2 (5% addition) | 154 |

Example 11

CSF Inducing Action on H-1/A Cells Differentiated into Adipocytes

$5.0 \times 10^6$ H-1/A cells were suspended in 50 ml of medium F and cultured at 33°C for 12 days in an Accel flask. During culturing, the medium was replaced with fresh medium F once every 4 days. After this time, the medium was replaced with medium E and culturing was continued for a further 3 days. As a result, oil droplets were observed to have accumulated in 40% or more of the cells, indicating differentiation into adipocytes. The medium was then replaced with medium E to which 5% of either culture supernatant from COS-1 cells transfected with pSR α-20-2, culture supernatant from COS-1 cells transfected with pcDL-SR α296 or serum-free DMEM had been added, and culturing was continued for 18 hours. After culturing, the medium was removed and the cells were washed twice with PBS(-), followed by addition of 25 ml of medium E to which sodium heparin had been added to a final concentration of 10 U/ml. This mixture was cultured at 33°C for 15 minutes. When LPL activity in the culture supernatant was measured, remarkable LPL suppression was only observed where culture supernatant from COS-1 cells transfected with pSR α-20-2 had been added (Table 8).

Table 8

| Additive | Relative Value of LPL Activity (%) |
|---|---|
| Serum-free DMEM (blank) | 100 |
| Culture supernatant of COS-1 cells transfected with pcDL-SR α296 (5% addition) | 80 |
| Culture supernatant of COS-1 cells transfected with pSR α-20-2 (5% addition) | 10 |

Subsequently, total RNA was isolated, in the same manner as in Example 1, from the cells prepared above. The amount of M-CSF mRNA was then analysed in the same manner as in Example 10, with the exception that 10 μg of total RNA was used each time. H-1/A cells treated with medium E containing 5% culture supernatant from COS-1 cells transfected with pSR α-20-2 demonstrated a remarkable increase in the amount of M-CSF mRNA per unit total RNA, when compared with H-1/A cells treated with medium E containing 5% culture supernatant from COS-1 cells transfected with pcDL-SR α296 (Table 9).

Table 9

| Additive | Relative Value of Amount of M-CSF mRNA (%) |
|---|---|
| Culture supernatant of COS-1 cells transfected with pcDL-SR α296 (5% addition) | 100 |
| Culture supernatant of COS-1 cells transfected with pSR α-20-2 (5% addition) | 172 |

Preparative Example 1

Enteric Coated Granule Preparation Containing 50% Adipogenesis Inhibitory Factor

70 parts (by weight - the same shall apply hereinbelow) of purified adipogenesis inhibitory factor obtained in Example 8, 10 parts of lactose and 20 parts of microcrystalline cellulose were weighed and thoroughly mixed in a mortar, followed by kneading with a suitable amount of water. The kneaded mixture was then extruded through a cylindrical granuliser, equipped with a screen having a pore size of 1.2 mm, to form granules. The granules were passed through a MARUMERIZER to provide a granular preparation. After these granules had been dried at 40°C for 2 hours in a circulating air dryer, the resulting dry granules were classified by passing through a 35 mesh sieve. Those granules larger than 35 mesh were used to manufacture the enteric coated granules. First, a solution of 5% hydroxypropylmethylcellulose, in equal amounts of methylene chloride and ethanol, was prepared according to conventional procedure, and 100 parts of the above-mentioned dry granules were spray-coated with 100 parts of this solution in a coating pan. Next, the thus treated granules were spray-coated in the same manner as described above with 300 parts of a solution of 10% hydroxypropylmethylcellulose phthalate (Shin-Etsu Chemical, HP-55), 1.5% stearic acid, 50% acetone and 38.5% ethanol prepared according to conventional procedure. The coated granules obtained in this manner were dried at 40°C for 1 hour in a circulating air dryer, to form an enteric preparation of adipogenesis inhibitory factor. These granules contain 50% adipogenesis inhibitory factor.

Preparative Example 2

Capsules

200 mg of the enteric coated granules prepared in Preparative Example 1 were charged into No. 3 capsules to produce hard capsules containing 100 mg of adipogenesis inhibitory factor per capsule.

Preparative Example 3

Injection

Adipogenesis inhibitory factor may be used in the form of an ampoule containing a sterile solution of the factor, dissolved or suspended in water or other pharmaceutically acceptable liquids. Otherwise, an ampoule containing a sterile powder preparation (preferably freeze-dried adipogenesis inhibitory factor) may be diluted at use with a pharmaceutically acceptable liquid.

[Sequence Listing]

(1) INFORMATION FOR

SEQ ID NO: 1

(i) SEQUENCE CHARACTERISTICS
Sequence length: 1065
Sequence type: Nucleic acid
Strandedness: double
Topology: Linear
Molecular type: cDNA to mRNA
Hypothetical: No
Anti-sense: No
Source:

Organism name: Homo sapiens
Cell line: KM-102

Sequence characteristics:
18-614 E CDS
81-614 E mat_peptide
18-80 E sig_peptide

(xi) SEQUENCE DESCRIPTION : SEQ ID NO: 1:

```
CCTGGCCCTG TGGGGAC ATG AAC TGT GTT TGC CGC CTG GTC    41
                   Met Asn Cys Val Cys Arg Leu Val
                   -21 -20                 -15

CTG GTC GTG CTG AGC CTG TGG CCA GAT ACA GCT GTC GCC   80
Leu Val Val Leu Ser Leu Trp Pro Asp Thr Ala Val Ala
            -10                 -5

CCT GGG CCA CCA CCT GGC CCC CCT CGA GTT TCC CCA GAC 119
Pro Gly Pro Pro Pro Gly Pro Pro Arg Val Ser Pro Asp
 1               5                  10

CCT CGG GCC GAG CTG GAC AGC ACC GTG CTC CTG ACC CGC 158
Pro Arg Ala Glu Leu Asp Ser Thr Val Leu Leu Thr Arg
    15                  20                  25
```

```
TCT CTC CTG GCG GAC ACG CGG CAG CTG GCT GCA CAG CTG 197
Ser Leu Leu Ala Asp Thr Arg Gln Leu Ala Ala Gln Leu
                 30                  35

AGG GAC AAA TTC CCA GCT GAC GGG GAC CAC AAC CTG GAT 236
Arg Asp Lys Phe Pro Ala Asp Gly Asp His Asn Leu Asp
 40                  45                  50

TCC CTG CCC ACC CTG GCC ATG AGT GCG GGG GCA CTG GGA 275
Ser Leu Pro Thr Leu Ala Met Ser Ala Gly Ala Leu Gly
         55                  60                  65

GCT CTA CAG CTC CCA GGT GTG CTG ACA AGG CTG CGA GCG 314
Ala Leu Gln Leu Pro Gly Val Leu Thr Arg Leu Arg Ala
                     70                  75

GAC CTA CTG TCC TAC CTG CGG CAC GTG CAG TGG CTG CGC 353
Asp Leu Leu Ser Tyr Leu Arg His Val Gln Trp Leu Arg
     80                  85                  90

CGG GCA GGT GGC TCT TCC CTG AAG ACC CTG GAG CCC GAG 392
Arg Ala Gly Gly Ser Ser Leu Lys Thr Leu Glu Pro Glu
                 95                 100

CTG GGC ACC CTG CAG GCC CGA CTG GAC CGG CTG CTG CGC 431
Leu Gly Thr Leu Gln Ala Arg Leu Asp Arg Leu Leu Arg
105                 110                 115

CGG CTG CAG CTC CTG ATG TCC CGC CTG GCC CTG CCC CAG 470
Arg Leu Gln Leu Leu Met Ser Arg Leu Ala Leu Pro Gln
        120                 125                 130

CCA CCC CCG GAC CCG CCG GCG CCC CCG CTG GCG CCC CCC 509
Pro Pro Pro Asp Pro Pro Ala Pro Pro leu Ala Pro Pro
                    135                 140
```

```
TCC TCA GCC TGG GGG GGC ATC AGG GCC GCC CAC GCC ATC 548
Ser Ser Ala Trp Gly Gly Ile Arg Ala Ala His Ala Ile
    145                 150                 155

CTG GGG GGG CTG CAC CTG ACA CTT GAC TGG GCC GTG AGG 587
Leu Gly Gly Leu His Leu Thr Leu Asp Trp Ala Val Arg
            160                 165

GGA CTG CTG CTG CTG AAG ACT CGG CTG TGACCCGGGG      624
Gly Leu Leu Leu Leu Lys Thr Arg Leu
170                 175         178

CCCAAAGCCA CCACCGTCCT TCCAAAGCCA GATCTTATTT ATTTATTTAT  674
TTCAGTACTG GGGGCGAAAC AGCCAGGTGA TCCCCCCGCC ATTATCTCCC  724
CCTAGTTAGA GACAGTCCTT CCGTGAGGCC TGGGGGACAT CTGTGCCTTA  774
TTTATACTTA TTTATTTCAG GAGCAGGGGT GGGAGGCAGG TGGACTCCTG  824
GGTCCCCGAG GAGGAGGGGA CTGGGGTCCC GGATTCTTGG GTCTCCAAGA  874
AGTCTGTCCA CAGACTTCTG CCCTGGCTCT TCCCCATCTA GGCCTGGGCA  924
GGAACATATA TTATTTATTT AAGCAATTAC TTTTCATGTT GGGGTGGGGA  974
CGGAGGGGAA AGGGAAGCCT GGGTTTTTGT ACAAAAATGT GAGAAACCTT 1024
TGTGAGACAG AGAACAGGGA ATTAAATGTG TCATACATAT C          1065
```

(2) INFORMATION FOR SEQ ID NO: 2:

(i) SEQUENCE CHARACTERISTICS:

Sequence length: 199
Sequence type: Amino acid
Topology: Linear
Molecular type: Protein
Hypothetical: No
Source:

Organism name: Homo sapiens
Cell line: KM-102

Sequence characteristics:

- 21--1 E sig peptide
1-178 E mat peptide

Sequence

Met Asn Cys Val Cys Arg Leu Val Leu Val Val Leu Ser Leu
-21 -20 -15 -10

Trp Pro Asp Thr Ala Val Ala Pro Gly Pro Pro Pro Gly Pro
-5 1 5

Pro Arg Val Ser Pro Asp Pro Arg Ala Glu Leu Asp Ser Thr
10 15 20

Val Leu Leu Thr Arg Ser Leu Leu Ala Asp Thr Arg Gln Leu
25 30 35

Ala Ala Gln Leu Arg Asp Lys Phe Pro Ala Asp Gly Asp His
40 45

Asn Leu Asp Ser Leu Pro Thr Leu Ala Met Ser Ala Gly Ala
50 55 60

Leu Gly Ala Leu Gln Leu Pro Gly Val Leu Thr Arg Leu Arg
65 70 75

Ala Asp Leu Leu Ser Tyr Leu Arg His Val Gln Trp Leu Arg
80 85 90

Arg Ala Gly Gly Ser Ser Leu Lys Thr Leu Glu Pro Glu Leu
95 100 105

Gly Thr Leu Gln Ala Arg Leu Asp Arg Leu Leu Arg Arg Leu
110 115

Gln Leu Leu Met Ser Arg Leu Ala Leu Pro Gln Pro Pro Pro
120 125 130

Asp Pro Pro Ala Pro Pro Leu Ala Pro Pro Ser Ser Ala Trp
135 140 145

Gly Gly Ile Arg Ala Ala His Ala Ile Leu Gly Gly Leu His
150 155 160

Leu Thr Leu Asp Trp Ala Val Arg Gly Leu Leu Leu Leu Lys
165 170 175

Thr Arg Leu
178

## Claims

1. A protein that includes no other human originating proteins and consisting of the amino acid sequence of amino acid numbers 1 to 178 of that indicated in SEQ. ID. NO. 2 of the sequence listing, or wherein one or more of the amino acid residues are deleted at one or more sites of said protein or wherein one of the amino acid residues is substituted at one of the sites of said protein, the protein being a 178 mer or part thereof, wherein the protein or part thereof possesses adipogenesis inhibitory activity.

2. A protein according to claim 1 consisting of the amino acid sequence of amino acid numbers 1 to 178 of that indicated in SEQ. ID. NO. 2 of the sequence listing and possesses adipogenesis inhibitory activity.

3. DNA coding for a protein according to either of Claims 1 or 2.

4. DNA coding for a protein according to either of Claims 1 or 2, consisting of a nucleotide sequence of nucleotide numbers 81 to 614 of that indicated in SEQ. ID. NO. 1 of the sequence listing.

5. A recombinant DNA expression vector comprising DNA according to either of Claims 3 and 4, wherein said DNA can be expressed and replicated.

6. A host cell transformed by a vector according to Claim 5.

**7.** A process for producing a protein, which comprises:

culturing a host cell according to Claim 6, and
recovering said protein from the cell extract or the culture solution.

**8.** A pharmaceutical composition containing a therapeutically effective amount of protein according to either of Claims 1 or 2, together with a pharmaceutically acceptable carrier or vehicle.

**9.** Use of a protein according to either of Claims 1 or 2, in the preparation of a medicament for the treatment or prophylaxis of cytopaenia.

**10.** Use of a protein according to either of Claims 1 or 2, in the preparation of a medicament for the treatment or prophylaxis of obesity.

**Patentansprüche**

**1.** Protein, das keine anderen Proteine aus dem Menschen enthält und aus der Aminosäuresequenz der Aminosäuren Nr. 1 bis 178 der SEQ ID Nr. 2 des Sequenzprotokolls besteht, oder worin ein oder mehrere der Aminosäurereste an einer oder mehreren Stellen des Proteins deletiert sind oder worin ein oder mehrere Aminosäurereste an einer Stelle des Proteins substituiert sind, wobei das Protein ein 178-mer oder Teil davon ist, wobei das Protein oder der Teil davon inhibitorische Aktivität gegen Fettbildung besitzt.

**2.** Protein nach Anspruch 1, das aus der Aminosäuresequenz der Aminosäuren Nr. 1 bis 178 der SEQ ID Nr. 2 des Sequenzprotokolls besteht und inhibitorische Aktivität gegen Fettbildung besitzt.

**3.** DNA, welche für ein Protein nach einem der Ansprüche 1 oder 2 kodiert.

**4.** DNA, welche für ein Protein nach einem der Ansprüche 1 oder 2 kodiert und aus einer Nukleotidsequenz der Nukleotide Nr. 81 bis 614 der SEQ ID Nr. 1 des Sequenzprotokolls besteht.

**5.** Expressionsvektor für rekombinante DNA, der DNA nach Anspruch 3 oder 4 enthält, worin die DNA exprimiert und repliziert werden kann.

**6.** Wirtszelle; die mit einem Vektor nach Anspruch 5 transformiert ist.

**7.** Verfahren zur Herstellung eines Proteins, welches umfasst:

Kultivieren einer Wirtszelle nach Anspruch 6 und Gewinnen des Proteins aus dem Zellextrakt oder der Kulturlösung.

**8.** Arzneimittelzusammensetzung, die eine therapeutisch wirksame Menge eines Proteins nach Anspruch 1 oder 2, zusammen mit einem pharmazeutisch geeigneten Träger oder Vehikel enthält.

**9.** Verwendung eines Proteins nach Anspruch 1 oder 2 zur Herstellung eines Arzneimittels für die Behandlung oder Prophylaxe von Cytopaenie.

**10.** Verwendung eines Proteins nach Anspruch 1 oder 2 zur Herstellung eines Arzneimittels für die Behandlung oder Prophylaxe von Fettleibigkeit.

**Revendications**

**1.** Protéine qui n'inclut aucune autre protéine d'origine humaine que et est constituée de la séquence d'acides aminés des acides aminés numéro 1 à 178 de la séquence SEQ. ID. N° 2 de la liste de séquences, ou dans laquelle un ou plusieurs résidus d'acide aminé sont supprimés au niveau d'un ou de plusieurs sites de ladite protéine ou dans laquelle l'un des résidus d'acide aminé est substitué au niveau de l'un des sites de ladite protéine, la protéine étant un 178mère ou une partie de celui-ci, la protéine ou une partie de celle-ci possédant une activité inhibitrice de l'adipogenèse.

2. Protéine selon la revendication 1, constituée de la séquence d'acides aminés des acides aminés numéro 1 à 178 de la séquence SEQ. ID. N° 2 de la liste de séquences et possédant une activité inhibitrice de l'adipogenèse.

3. ADN codant une protéine selon l'une quelconque des revendications 1 ou 2.

4. ADN codant une protéine selon l'une quelconque des revendications 1 ou 2, constituée d'une séquence nucléotidique des nucléotides numéro 81 à 614 de la séquence SEQ. ID. N° 1 de la liste de séquences.

5. Vecteur d'expression d'ADN recombinant comprenant un ADN selon l'une quelconque des revendications 3 et 4, dans lequel ledit ADN peut être exprimé et répliqué.

6. Cellule hôte transformée par un vecteur selon la revendication 5.

7. Procédé de production d'une protéine qui comprend : la culture d'une cellule hôte selon la revendication 6 et la récupération de ladite protéine à partir de l'extrait cellulaire ou de la solution de culture.

8. Composition pharmaceutique contenant une quantité thérapeutiquement efficace d'une protéine selon l'une quelconque des revendications 1 ou 2, avec un support ou un véhicule pharmaceutiquement acceptable.

9. Utilisation d'une protéine selon l'une quelconque des revendications 1 ou 2, pour la préparation d'un médicament destiné au traitement ou la prophylaxie d'une cytopénie.

10. Utilisation d'une protéine selon l'une quelconque des revendications 1 ou 2, pour la préparation d'un médicament destiné au traitement ou la prophylaxie de l'obésité.

FIG.1.

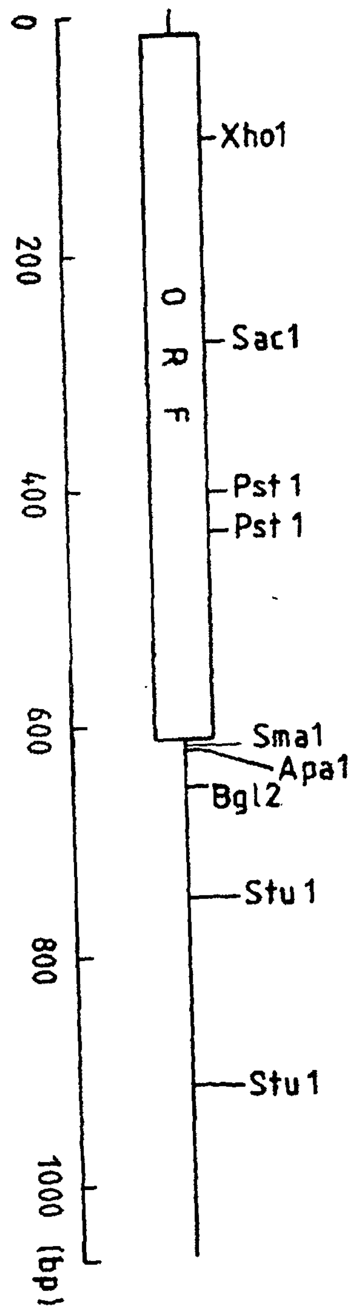
0
200
400
600
800
1000 (bp)
O R F
Xho1
Sac1
Pst 1
Pst 1
Sma1
Apa1
Bgl2
Stu 1
Stu 1

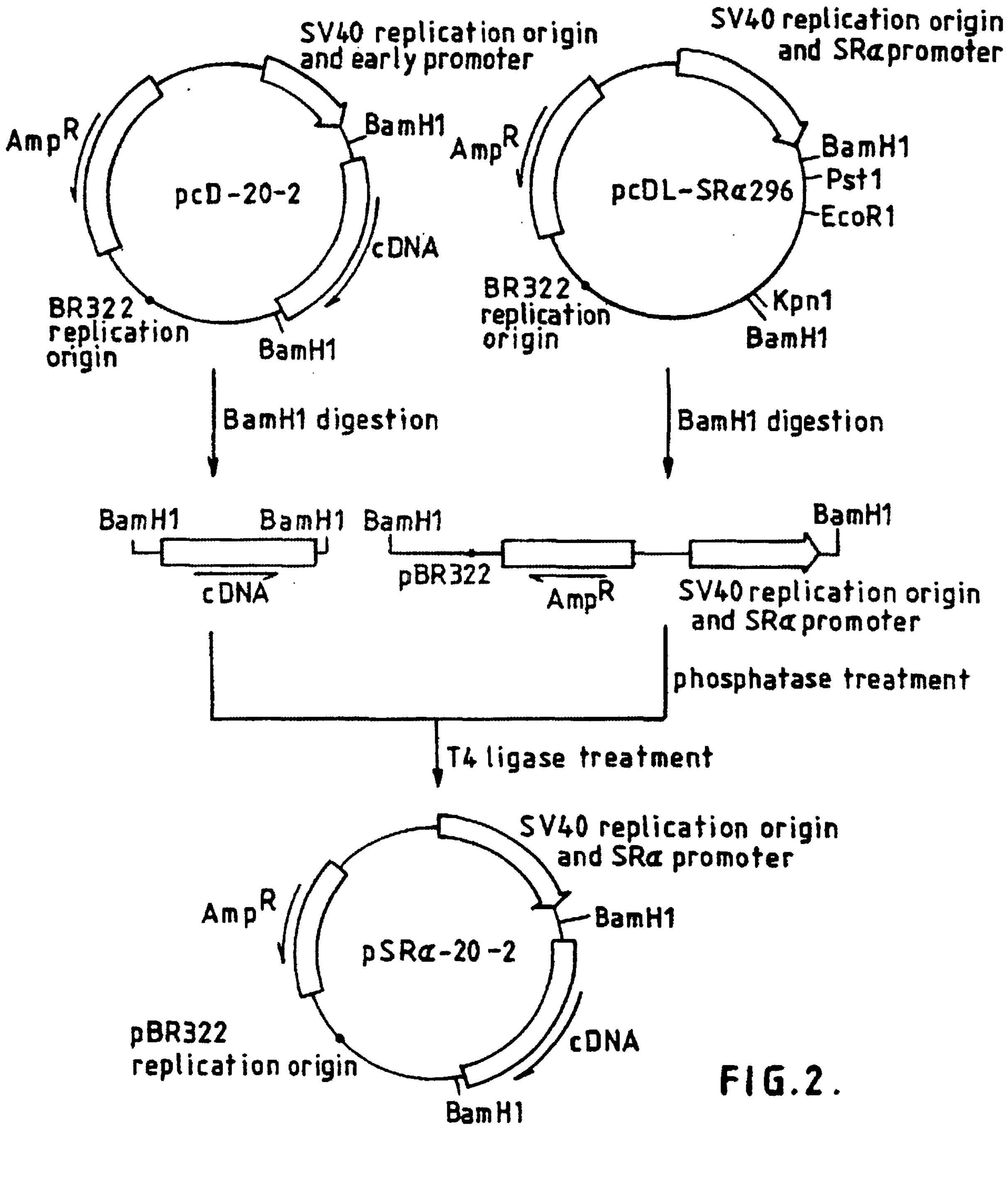
SV40 replication origin
and early promoter
Amp$^R$
BamH1
pcD-20-2
cDNA
BR322
replication
origin
BamH1
SV40 replication origin
and SRα promoter
Amp$^R$
BamH1
Pst1
pcDL-SRα296
EcoR1
BR322
replication
origin
Kpn1
BamH1
BamH1 digestion
BamH1 digestion
BamH1
BamH1
BamH1
BamH1
cDNA
pBR322
Amp$^R$
SV40 replication origin
and SRα promoter
phosphatase treatment
T4 ligase treatment
SV40 replication origin
and SRα promoter
Amp$^R$
BamH1
pSRα-20-2
pBR322
replication origin
cDNA
BamH1

FIG.2.

FIG.3.

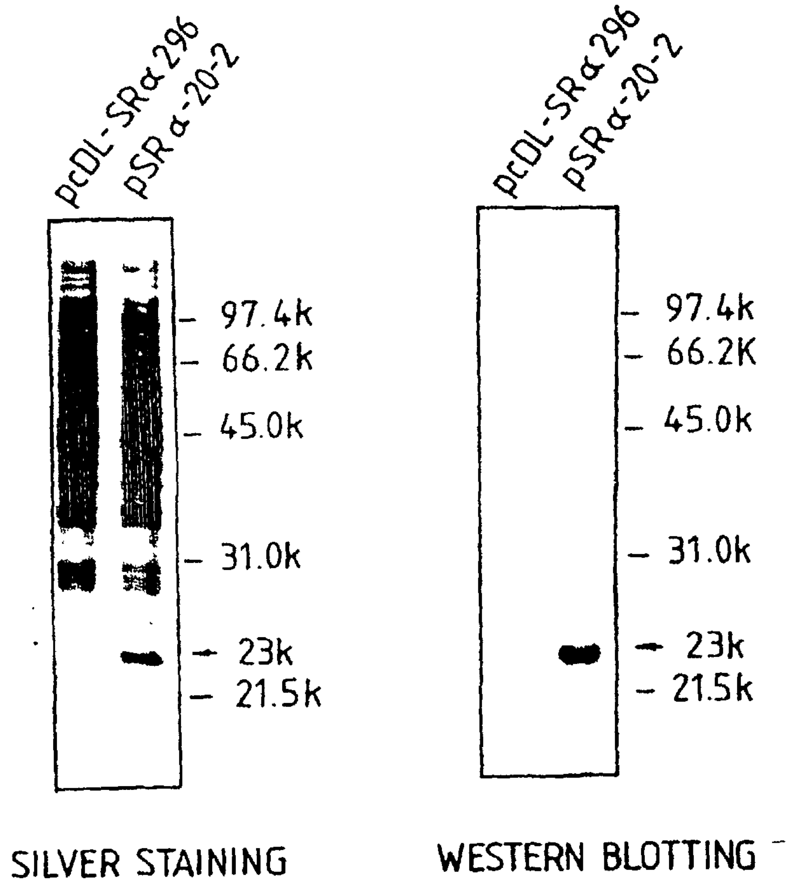
pcDL-SRα296
pSRα-20-2
97.4k
66.2k
45.0k
31.0k
23k
21.5k
SILVER STAINING
pcDL-SRα296
pSRα-20-2
97.4k
66.2K
45.0k
31.0k
23k
21.5k
WESTERN BLOTTING